# EUROPÄISCHE PATENTSCHRIFT

(11) **EP 2 157 067 B1**
(45) Veröffentlichungstag und Bekanntmachung des Hinweises auf die Patenterteilung: **12.09.2012**
(21) Anmeldenummer: 08161501.5
(22) Anmeldetag: 30.07.2008
(51) Int. Cl.: C04B 35/628, C04B 35/488, A61K 6/06, A61C 13/00, C04B 35/626, A61C 13/083

(54) **Mit einer farbgebenden Komponente beschichtete Primärpartikel**
Primary particle coated with a colouring component
Particule primaire revêtue d'un agent colorant

(43) Veröffentlichungstag der Anmeldung: 24.02.2010
(73) Patentinhaber: Ivoclar Vivadent AG, 9494 Schaan (LI)
(72) Erfinder: Apel, Elke, 9479 Oberschan (CH); Ritzberger, Christian, 6710 Nenzing (AT); Höland, Wolfram, 9494 Schaan (LI); Appert, Christoph, 9490 Vaduz (LI); Wachter, Wolfgang, 9494 Schaan (LI); Rheinberger, Volker, 9490 Vaduz (LI)
(74) Vertreter: UEXKÜLL & STOLBERG

(56) Entgegenhaltungen:
- EP-A- 0 535 796
- EP-A- 1 859 757
- EP-A1- 0 329 565
- EP-A1- 0 401 045
- WO-A-2008/023053
- DE-A1- 19 950 284
- DE-A1-102005 003 755
- DE-A1-102006 015 014
- GB-A- 421 872
- JP-A- 1 009 839
- US-A- 5 496 682

## Beschreibung

Die Erfindung betrifft eine Suspension auf Basis von Oxidkeramikmaterial, die mit einer farbgebenden Komponente beschichtete Primärpartikel enthält, ein Verfahren zu deren Herstellung und ihre Verwendung, insbesondere in der Herstellung von Keramikformteilen und Dentalrestaurationen. Die Erfindung betrifft ferner ein Verfahren zur Herstellung eines Keramikformteils.

In der restaurativen Zahnmedizin haben Hochleistungskeramiken, wie Al₂O₃ und tetragonales ZrO₂, große Bedeutung zur Fertigung von weitspannigen Brücken und hochbelastbaren Kronen erlangt. Im Folgenden wird hauptsächlich auf die Verarbeitung von ZrO₂-Materialien eingegangen. Die Herstellung dieser DentalRestaurationen erfolgt dabei bevorzugt durch maschinelle Bearbeitung von ZrO₂-Rohlingen, die aus granulierten ZrO₂-Pulvern durch Verpressen und in den meisten Fällen nachfolgende thermische Behandlung erzeugt werden.

Aus dem Stand der Technik sind verschiedene Methoden bekannt, um Oxidkeramik-Pulver so einzufärben, dass das daraus hergestellte Formteil die angestrebte Farbe aufweist.

Die wohl bekannteste Methode ist das Beimischen von färbenden Oxiden in das Granulat der Oxidkeramik. Nach dessen Verpressen, Verarbeiten und der thermischen Bearbeitung entsteht dann das fertige eingefärbte Formteil. Durch die thermische Behandlung belegen die färbenden Ionen einen Gitterplatz oder einen Zwischengitterplatz. Die wichtigsten Dokumente hierfür sind: US 5,219,805 bzw. US 5,263,858, US 5,656,564 oder US 5,059,562 bzw. US 5,118,457.

Zur Herstellung von farbigen Keramikformteilen, insbesondere von Dentalformteilen, deren Färbung derjenigen der natürlichen Zähne möglichst nahe kommt, sind weitere Ansätze vorgeschlagen worden. So sind aus dem Stand der Technik Verfahren bekannt, nach denen farbige Rohlinge oder auch dentale Restaurationsteile durch Infiltration von Flüssigkeiten in einen vorgesinterten Formkörper erhalten werden (US 6,709,694 bzw. EP 1 486 476). Diese Verfahren haben jedoch den Nachteil, dass eine Farbgebung nach dem Vorsinterprozess erfolgt und damit in einen offenporigen Keramikkörper Flüssigkeiten eingebracht werden. Damit ist die Farbgebung nicht vollständig homogen. Auch eine Mehrfarbigkeit ist nicht erzielbar. Insbesondere können durch die nachträgliche Einfärbung eines teilgesinterten Rohlings oder eines geformten Dentalproduktes nur die Hohlräume (Poren) zwischen den teilweise zusammengesinterten Partikeln des Ausgangspulvers durch die färbenden Materialien belegt werden. Somit werden auch nur diskrete Bereiche der Oberfläche der Partikel mit einer Schicht der färbenden Oxide gefärbt, eine kontinuierliche Belegung der Oberfläche der Partikel des Ausgangspulvers ist hingegen nicht möglich. Ein weiterer großer Nachteil bei einer Infiltration besteht im Konzentrationsgradienten der Einfärbung von außen nach innen. Wird ein poröser Körper in die Färbelösung eingelegt, so gibt die Ausgangslösung beim Eindringen in den Körper einen Teil der gelösten färbenden Ionen ab und "verarmt" so von außen nach innen an färbenden Substanzen. Die Konsequenz daraus ist, dass außen eine höhere Konzentration der färbenden Ionen bzw. Oxiden vorliegt als im Inneren des Formkörpers. Weiterhin kann mittels der Infiltrationstechnik nur eine bestimmte Eindringtiefe erreicht werden.

EP 1 859 757 A2 beschreibt ein Verfahren zur Herstellung von gefärbten Rohlingen und dentalen Formteilen. Dabei werden Oxidkeramik-Pulver als Granulat in einem Wirbelschichtreaktor mit einer farbgebenden Substanz in wässriger Lösung beschichtet. Die so erhaltenen beschichteten Granulate werden anschließend zu einem Formkörper verpresst, der nach einer Vorsinterung durch Fräsen oder Schleifen zu einem dentalen Formteil weiterverarbeitet werden kann.

Die durch Pressverfahren hergestellten Formkörper werden üblicherweise in Form von Blockkörpern erhalten, aus denen beispielsweise mittels der CAD/CAM-Technologie die gewünschten Restaurationen herausgefräst werden können. Solche Blockkörper, auch mit einem kontinuierlichen Farbverlauf, sind etwa in der EP 1 859 758 A1 beschrieben. Nachteilig an dieser Technologie ist insbesondere der mit dem Fräsverfahren einhergehende erhebliche Verlust an wertvoller Sinterkeramik.

Neben den trockenen Pressverfahren ist im Stand der Technik auch die Verwendung von Keramikpartikeln in Suspensionen, insbesondere in Form sogenannter Schlicker, bekannt. Hierbei kann es vorteilhaft sein, auf die Keramikpartikel eine Beschichtung aufzubringen, die beispielsweise als Verarbeitungshilfsmittel dienen kann. So beschreibt DE 10 2005 003 755 A1 ein Verfahren zur Beschichtung eines Dentalpulvers mit anorganischen Stoffen, wie Brønsted- oder Lewis-Säuren oder Basen, oder organischen Stoffen, insbesondere bestimmten Polymeren. Die Beschichtung erfolgt jeweils unter Verwendung einer wässrigen Lösung der Beschichtungskomponente entweder direkt durch Einbringung des Pulvers in das wässrige Medium oder durch ein Wirbelschichtverfahren.

Weiterhin ist aus der EP 1 210 054 ein Verfahren bekannt, nach dem gefärbte Rohlinge aus teilstabilisiertem Zirkondioxid so hergestellt werden, dass die Ausgangsmaterialien in Form ihrer löslichen Chloride einschließlich der färbenden Substanzen in Wasser gelöst werden, eine Kofällung durchgeführt wird und das Fällungsprodukt bei ca. 700 °C kalziniert wird. Nach der Mahlung des Kalzinates sowie einer Sprühtrocknung wird das so erhaltene Granulat isostatisch verpresst und anschliessend thermisch behandelt (Entbinderung und Vorsinterung).

In der Dentaltechnik ist die bis heute gebräuchlichste Methode das Schleifen bzw. Fräsen von Blöcken, Platten oder Zylindern. Die Entscheidung, welche Art der mechanischen Bearbeitung gewählt wird, hängt von den jeweiligen Maschinen, aber auch vom Zustand der Oxidkeramik (Verhältnis der Dichte des zu bearbeitenden Teiles zur theoretisch erreichbaren Dichte) ab.

Einen weiteren Ansatz zur Bildung von oxidkeramischen Formteilen stellen die sogenannten aufbauenden oder generativen Fertigungsverfahren dar. Dabei werden unter dem Begriff "Rapid Prototyping" (RP) generative Fertigungsverfahren zusammengefasst, bei denen aus computergestützten Konstruktionsdaten (CAD-Daten) 3-dimensionale Modelle oder Bauteile hergestellt werden (A. Gebhardt, Vision of Rapid Prototyping, Ber. DGK 83 (2006) 7-12). Dabei handelt es sich um Verfahren, wie z.B. Stereolithographie (SL), selektives Lasersintern (SLS), 3-D-Printing, Fused Deposition Modelling (FDM), Ink-Jet-Printing (IJP), 3D-Plotting, Multi-Jet Modelling (MJM), Solid Freeform Fabrication (SFF), Laminated Object Manufacturing (LOM), Laser Powder Forming (LPF) und Direct Ceramic Jet Printing (DCJP), mit denen sich Modelle, Bau- oder Formteile auch in Kleinserie kostengünstig herstellen lassen (A. Gebhardt, Generative Fertigungsverfahren, 3. Aufl., Carl Hanser Verlag, München 2007, 77ff.). Bei der Stereolithographie handelt es sich um RP-Verfahren (A. Beil, Fertigung von Mikro-Bauteilen mittels Stereolithographie, Düsseldorf 2002, VDI-Verlag 3 ff.), bei denen ein Formteil auf der Basis von CAD-Daten schichtweise aus einem flüssigen und härtbaren Monomerharz aufgebaut wird.

RP-Verfahren zur Herstellung von dentalen Formkörpern, wie Inlays, Kronen oder Brücken, sind vor allem bei keramischen Werkstoffen von großem Vorteil, da damit eine deutliche Vereinfachung der im zahntechnischen Labor mit großem manuellen Aufwand betriebenen Abform- und Gießprozesse bzw. der Fräs-und Schleifoperationen möglich ist und gleichzeitig der bei nicht-generativen Verfahren auftretende Materialverlust vermieden werden kann. Da heutzutage eine vollständige digitale Prozesskette etabliert ist, lassen sich die klassischen Verfahrensschritte zur Herstellung von z.B. mehrgliedrigen Brükkengerüsten (Ausrichten im Artikulator, Wachsmodulation, Einbetten und Guss) durch die Digitalisierung des Modells, virtuelle Konstruktion des dentalen Formkörpers und dessen generative stereolithographische Fertigung ersetzen.

Als wichtige Methoden zur Herstellung dentaler Formteile aus oxidkeramischen Materialien haben sich sowohl die Stereolithographie und zunehmend auch das 3D-Printing erwiesen. Für das 3D-Printing werden sprühfähige keramische Tinten verwendet, die oxidkeramische Partikel in einem vernetzbaren Lösungsmittel enthalten. Nach dem Auftragen einer Schicht erfolgt die Aushärtung durch energiereiche Strahlung. Im Gegensatz dazu wird bei der Stereolithographie eine Schicht aus einem vernetzbaren Schlicker durch gezielte Belichtung ausgehärtet.

Die Schlicker und keramischen Tinten weisen eine im Wesentlichen übereinstimmende Zusammensetzung auf, zumindest hinsichtlich der verwendeten Komponenten. Diese sind in beiden Fällen die oxidkeramischen Partikel, ein vernetzbares Monomer bzw. Monomerengemisch, ein Initiator bzw. Initiatorsystem und ggf. weitere Hilfsmittel wie Lösungsmittel etc.

Bei der Herstellung von keramischen Formteilen z.B. mittels Stereolithographie wird zunächst ein keramischer Grünling durch schichtweise Härtung eines fließfähigen keramischen Schlickers hergestellt, der dann nach der Entbinderung zu einem dichten keramischen Formkörper gesintert wird. Der Grünling wird auch Grünkörper genannt. Als Entbinderung wird das Austreiben des Bindemittels bezeichnet. Hierbei wird das verwendete Bindemittel üblicherweise durch Erhitzen des Grünlings auf eine Temperatur von ca. 90 °C bis 600 °C entfernt. Wesentlich ist, dass die Bildung von Rissen und Deformationen weitestgehend vermieden wird. Durch die Entbinderung wird aus dem Grünling der sogenannte Weissling.

Beim Entbindern finden rein thermische wie auch thermochemische Prozesse statt. Üblicherweise werden beim Verpressen von keramischen Pulvern als Binder Mischungen von Wasser, Lösungsmitteln, Polymeren, Wachsen oder Ölen eingesetzt. Als Polymere finden meist Polypropylen, Polyethylen, Polyvinylacetat, Polyvinylalkohol, Methylcellulose, Polyvinylpyrrolidon, Polystyrol oder Polyethylmethacrylat Anwendung (vgl. R. Moreno, Amer. Cer. Soc. Bull. 71 (1992) 1647-1657). Hierbei handelt es sich um lineare Polymere, die durch Depolymerisation oder Kettenspaltung bei höherer Temperatur mehr oder weniger leicht zu flüchtigen Komponenten abgebaut werden.

Im Falle mittels RP-Verfahren erzeugter Grünlinge auf der Basis von vernetzenden Monomermischungen liegt ein Polymernetzwerk vor. Durch die Verwendung vernetzender Monomere kann die Härtungszeit, die erforderlich ist, um einen stabilen Festkörper zu erhalten, deutlich verkürzt werden, gleichzeitig weist das sich ausbildende Polymernetzwerk im Vergleich zu linearen Polymeren aber auch eine deutlich erhöhte thermische Stabilität auf, was den Entbinderungsprozess nachteilig beeinflusst.

Die Sinterung des Weisslings erfolgt im Sinterofen beim Hochtemperaturbrand. Dabei kommt es zur Verdichtung und Verfestigung des fein verteilten Keramikpulvers durch Temperatureinwirkung unterhalb der Schmelztemperatur der Hauptkomponenten, wodurch das poröse Bauteil kleiner wird und dessen Festigkeit zunimmt.

Die US 5,496,682 offenbart lichthärtbare Zusammensetzungen für die Herstellung dreidimensionaler Körper durch Stereolithographie, die 40 bis 70 Vol.-% Keramik- oder Metallpartikel, 10 bis 35 Gew.-% Monomer, 1 bis 10 Gew.-% Photoinitiator, 1 bis 10 Gew.-% Dispergiermittel und vorzugsweise auch Lösungsmittel, Weichmacher und Kopplungsmittel enthalten.

DE 10 2006 015 014 A1 beschreibt ein Verfahren zur Herstellung dreidimensionaler keramischer Formkörper durch lagenweises Aufdrucken einer Suspension mit Hilfe eines Tintenstrahldrukkers. Die Suspension enthält Keramikteilchen in einem Dispergiermedium auf Basis eines wässrigen Böhmitsols.

Die US 6,117,612 offenbart Harze für die stereolithographische Herstellung von gesinterten Keramik- oder Metallteilen. Die Harze haben eine Viskosität von weniger als 3000 mPa•s. Zur ihrer Herstellung werden Monomere mit geringer Viskosität eingesetzt, vorzugsweise in wässriger Lösung. Durch die Verwendung von Dispersionsmitteln soll ein hoher Feststoffgehalt bei geringer Viskosität erzielt werden.

Die DE 199 50 284 A1 beschreibt mit sichtbarem Licht aushärtbare Zusammensetzungen auf Basis von polymerisierbaren Monomeren oder Oligomeren und deren Verwendung zur Herstellung von Dentalrestaurationen aus Kunststoffmaterialien mit RP-Verfahren.

Ein besonderes Problem bei der Herstellung von Keramikformteilen durch RP-Verfahren ist die Farbgebung der Keramik, da die verwendeten Färbemittel den Entbinderungs- und Sinterprozess überstehen müssen. Zudem hat sich gezeigt, dass es bei Verwendung von Pigmenten, d.h. überwiegend kristallinen anorganischen Stoffen, zur Farbgebung von Keramiken häufig zu inselartigen Anhäufungen der Pigmente in der Keramik kommt. Durch diese inhomogene Verteilung der Pigmente werden zum einen nicht die gewünschten Farbeffekte erzielt, sondern darüber hinaus auch die Lichtdurchlässigkeit der Keramik beeinträchtigt. Zudem führt die hohe lokale Konzentration von Fremdmaterial in der Keramik zu einer Minderung der Festigkeit.

Die oben beschriebenen Verfahren sind insbesondere nicht ausreichend geeignet, um unter Verwendung generativer Verfahren farbige Keramikformteile aufzubauen, die den Anforderungen an Dentalmaterialien genügen. Der Erfindung liegt daher die Aufgabe zugrunde, eine verbesserte Technologie zur Herstellung von farbigen Keramikformteilen insbesondere mittels RP-Verfahren zur Verfügung zu stellen.

Diese Aufgabe wird erfindungsgemäß durch eine Suspension auf Basis von Oxidkeramikmaterial gelöst, die
(A) 10 - 95 Gew.-% Primärpartikel von Oxidkeramikmaterial, wobei die Primärpartikel eine durchschnittliche Partikelgröße im Bereich von 10 bis 1000 nm aufweisen und mit einer farbgebenden Komponente beschichtet sind und wobei die Primärpartikel bezogen auf die Gesamtmasse der Primärpartikel 0,000001 bis 0,4 Gew.-% der farbgebenden Komponente enthalten,
(B) 3 - 85 Gew.-% polyreaktives Bindemittel,
(C) 1 - 80 Gew.-% organisches Lösungsmittel und
(D) 1 bis 30 Gew.-% weitere Additive enthält,
jeweils bezogen auf die Gesamtmasse der Suspension. Der Begriff mittlere Partikelgröße bezieht sich hier auf das Zahlenmittel.

Die in der erfindungsgemäßen Suspension enthaltenen Primärpartikel sind überraschenderweise dazu geeignet, bei ihrer Verwendung in der Herstellung von Keramikformteilen eine optimal homogene Färbung des erhaltenen Formteils zu erzielen.

Dies stellt einen wesentlichen Vorteil gegenüber den im Stand der Technik verwendeten Keramikmaterialien dar. Die so erhaltene Farbhomogenität ist sogar besser als die Farbhomogenität, die durch Verwendung von mit farbgebenden Komponenten beschichteten Agglomeraten und Granulaten erhalten wird. Aufgrund der hohen Homogenität der Einfärbung wird dabei eine optimale Farbwirkung bereits bei Verwendung sehr geringer Mengen an farbgebenden Komponenten erreicht. Insbesondere ist es nicht notwendig, mit einem Überschuss an farbgebenden Ionen zu arbeiten. Die im Vergleich zum Stand der Technik geringere benötigte Menge an farbgebenden Verbindungen stellt angesichts hoher Preise für diese Verbindungen einen weiteren Vorteil der Erfindung dar. Außerdem wird durch die größere Homogenität die Zahl von Fehlstellen in der Keramik verringert, was sich positiv auf deren Festigkeit auswirkt.

Erfindungsgemäß werden unter Primärpartikeln oxidkeramische Partikel verstanden, die eine mittlere Partikelgröße im Bereich von 10 bis 1000 nm aufweisen (d₅₀ = 10 bis 1000 nm). Die mittlere Partikelgröße liegt vorzugsweise im Bereich von 100 bis 200 nm. Diese Partikel werden mit einer farbgebenden Komponente beschichtet.

Die chemische Zusammensetzung der verwendeten oxidkeramischen Pulver umfasst vorzugsweise ZrO₂- oder Al₂O₃-Pulver oder Mischungen beider Oxide. Besonders bevorzugt wird ZrO₂, Al₂O₃, CaO-, CeO₂-, und/oder MgO-stabilisiertes ZrO₂, insbesondere yttriumstabilisiertes Zirkonoxid eingesetzt. Ganz besonders bevorzugt ist die Verwendung von 3Y-TZP (Yttrium Stabilized Tetragonal Zirconium Dioxide Polycrystals), d.h. ZrO₂, das mit 3 Mol% Y₂O₃ stabilisiert ist.

Zur Beschichtung der Primärpartikel werden diese zuerst in einem Trägermedium dispergiert und mit der farbgebenden Komponente, die in diesem Trägermedium löslich sein muss, vermischt.

Die oxidkeramischen Primärpartikel können in organischen Suspensionsmedien dispergiert und weiterverarbeitet werden. Durch die homogene Verteilung der farbgebenden Komponenten in den so erhaltenen organischen Suspensionen ist es möglich, eine homogene Verteilung der farbgebenden Ionen auf der Oberfläche der Primärpartikel zu erreichen, die schließlich in einer homogenen Färbung der Keramik nach der Sinterung resultiert.

Als farbgebende Komponente wird vorzugsweise eine metallorganische Verbindung verwendet, wie z.B. ein Acetylacetonat oder ein Carbonsäuresalz, die in dem verwendeten organischen Suspensionsmedium löslich ist.

Die Metallionen in diesen metallorganischen Verbindungen gehören zur Gruppe der Übergangsmetalle. Bevorzugt sind Verbindungen der Elemente Eisen, Cer, Praseodym, Terbium oder Mangan.

Als farbgebende Komponente kann sowohl eine einzelne metallorganische Verbindung als auch eine Kombination von mehreren Verbindungen eingesetzt werden, so dass entweder nur ein (1) Übergangsmetall oder eine Kombination von mehreren Metallen die spezifische Färbung der Oxidkeramik hervorruft.

Für die organischen Suspensionen sind Salze der Carbonsäuren, Essig-, Propion, Butter-, 2-Ethylhexylcarbon-, Stearin- und Palmitinsäure bevorzugt. Besonders bevorzugt sind vor allem die entsprechenden Fe-, Pr-, Mn- und Tb-Verbindungen wie z.B. Eisen(III)-acetat oder -acetylacetonat, Praseodym(III)-acetat oder -acetylacetonat oder Terbium(III)-acetat oder -acetylacetonat sowie die entsprechenden Carbonsäuresalze.

Die Weiterverarbeitung der organisch-basierten keramischen Suspension geschieht vorzugsweise durch aufbauende RP-Verfahren wie z.B. Stereolithographie oder 3D-Printing. Mit diesen Verfahren werden keramische Formteile schichtenweise aufgebaut.

Die Beschichtung der Primärpartikel erfolgt z.B. auf die in der EP 1 859 757 A2 beschriebene Weise, wobei dort jedoch keine Beschichtung von Primärpartikeln erfolgt, sondern eine Beschichtung von bereits vorgefertigten, pressfähigen Granulaten durchgeführt wird.

Aus der fertigen Suspension können beschichtete Primärpartikel beispielsweise dadurch gewonnen werden, dass das Trägermedium durch Verdampfung schonend entfernt wird. Sofern dabei die beschichteten Primärpartikel zunächst in Form von Aggregaten oder Agglomeraten anfallen, können diese etwa durch ein übliches Zerkleinerungsverfahren zerstört und so die beschichteten Primärpartikel erhalten werden. Vorzugsweise kann die fertige Suspension jedoch direkt wie nachfolgend beschrieben in Weiterverarbeitungsverfahren eingesetzt werden.

Die beschriebenen Suspensionen, die gleichzeitig Primärpartikel und die farbgebende Komponente enthalten, werden z.B. mittels Sprühgranulierung zu pressfähigen Granulaten weiterverarbeitet. Durch die Behandlung der Primärpartikel mit der farbgebenden Komponente bereits vor dem Granulierungsschritt wird eine homogene Verteilung der färbenden Ionen auf der Oberfläche der Primärpartikel erreicht. Nach der Granulierung, die einen Agglomerationsprozess der Primärpartikel darstellt, sind die farbgebenden Ionen homogener als bisher in den einzelnen Granulatpartikeln verteilt. Dies stellt eine wesentliche Verbesserung gegenüber dem Stand der Technik dar.

Die Primärpartikel können nach der Granulierung zu Formteilen, insbesondere zu Blöcken, weiterverarbeitet werden, vorzugsweise mittels Trockenpressverfahren. Nach dem Vorsintern dieser oxidkeramischen Formteile können diese durch CAD/CAM-Technologie zu dentalen Kronen und Brückengerüsten weiterverarbeitet werden.

Erfindungsgemäß enthalten die Primärpartikel nach der Beschichtung 0,000001 bis 0,4 Gew.-%, insbesondere 0,0001 bis 0,3 Gew.-%, besonders bevorzugt 0,01 bis 0,2 Gew.-%, farbgebende Komponente, bezogen auf die Gesamtmasse der Partikel. Dabei beziehen sich diese Werte unabhängig vom Typ der verwendeten Übergangsmetallverbindungen (organisch oder anorganisch) immer auf die entsprechenden Übergangsmetalloxide. Beispielsweise wird bei Verwendung von Eisen(III)-acetat oder -acetylacetonat als farbgebende Komponente der entsprechende rechnerische Gewichtsanteil an Fe₂O₃ angegeben.

Vorzugsweise wird die farbgebende Komponente so gewählt, dass nach einem Sinterprozess zahnfarbene Keramikformteile erhalten werden. Dazu können insbesondere zwei oder mehr der oben genannten Übergangsmetallverbindungen zur Erzielung einer speziellen Farbnuance kombiniert werden.

Die organischen Suspensionen werden vorzugsweise zur Herstellung von oxidkeramischen Formteilen mittels Rapid-Prototyping-Verfahren, wie z.B. Stereolithographie, 3D-Printing oder selektives Lasersintern, verwendet. Der Vorteil dieser aufbauenden Verfahren gegenüber spanabhebenden Technologien ist der geringere Einsatz an hochwertigem oxidkeramischem Material. In den RP-Verfahren wird nur so viel Material eingesetzt, wie es für das spezifische keramische Formteil erforderlich ist. Im Unterschied dazu ist bei spanabhebenden Technologien der Materialverbrauch höher, da die spezifische Geometrie des dentalen Bauteils aus einem keramischen Grundkörper (Block, Platte, Zylinder) herausgearbeitet wird und somit ein deutlich höherer Materialverbrauch gegeben ist.

Die keramischen Formteile, die mittels RP-Verfahren hergestellt wurden, bestehen aus einer stabilen organischen Matrix, in der die oxidkeramischen Primärpartikel homogen verteilt und eingebettet sind. Die organische Matrix muss eine solche Mindestfestigkeit besitzen, dass die erforderliche Formstabilität der Bauteile bei der Weiterverarbeitung gewährleistet ist.

Erst während der Sinterung werden die Übergangsmetallionen in das Kristallgitter z.B. der ZrO₂-Keramik eingebaut, woraus die Färbung der Keramik resultiert. Das gilt für alle Prozesse unabhängig vom Typ der Suspensionen und unabhängig vom Typ des verwendeten technologischen Verfahrens (RP-Verfahren, Sprühgranulierung, Gießverfahren).

Um eine homogene Verteilung der Partikel im Trägermedium zu erreichen und um Partikelaggregate und -agglomerate zu zerstören, wird das oxidkeramische Pulver vor der Behandlung mit der farbgebenden Komponente vorzugsweise einem Homogenisierungsund Zerkleinerungsprozess unterzogen. Dabei kann es sich um einen Mahlprozess in einer für keramische Pulver üblichen Mühle oder um einen Dispergierprozess mittels Ultraschall handeln. Bei diesen Prozess kommen z.B. Mühlen, insbesondere Rührwerkskugelmühlen, Dissolver oder Ultraschall-Dispergatoren zum Einsatz.

Der Homogenisierungs- oder Zerkleinerungsprozess wird vorzugsweise in Anwesenheit des gewünschten Suspensionsmediums durchgeführt. Während dieses Prozesses wird die Suspension mit den Komponenten zur Farbgebung und ggf. zur Stabilisierung versetzt und im Verlauf des Prozesses zerkleinert und homogenisiert. Die fertige Suspension wird dann wie oben beschrieben in Weiterverarbeitungsverfahren (RP-Verfahren, Sprühgranulierung) eingesetzt.

Die beschriebenen Primärpartikel sind generell zur Verwendung in verschiedensten Verfahren zur Herstellung von Keramikformteilen und insbesondere Dentalrestaurationen, wie z.B. Inlays, Onlays, Veneers, Kronen, Brücken oder Gerüsten geeignet, beispielsweise durch trockenes Verpressen und nachfolgendes Sintern. In diesen Verfahren werden die behandelten Primärpartikel vorzugsweise in Form eines Granulats eingesetzt, das die Primärpartikel enthält. Geeignete Granulate können insbesondere durch die oben beschriebene Sprühgranulierung erhalten werden.

Beispiele für weitere bevorzugte Verfahren sind Rapid Prototyping (RP)-Verfahren, wie Stereolithographie, 3D-Printing oder selektives Lasersintern. Diese Verfahren sind besonders vorteilhaft, weil sie als nicht-spanabhebende Technologien den mit spanabhebenden Technologien einhergehenden Materialverlust vermeiden. Zur Verwendung in diesen Technologien werden die Primärpartikel typischerweise in Form einer erfindungsgemäßen flüssigen Suspension, insbesondere eines flüssigen organischen Schlickers oder einer keramischen Tinte eingesetzt.

Bevorzugt ist eine Suspension auf Basis von Oxidkeramikmaterial, die 40 - 90 Gew.-%, besonders bevorzugt 70 - 85 Gew.-% Primärpartikel und 7 - 80 Gew.-%, am meisten bevorzugt 10 - 20 Gew.-% organische Komponente enthält, jeweils bezogen auf die Gesamtmasse der Suspension.

Besonders bevorzugt ist eine Suspension, die
(A) 40 - 90 Gew.-%, besonders bevorzugt 70 - 85 Gew.-% Primärpartikel,
(B) 5 - 40 Gew.-%, besonders bevorzugt 7 - 15 Gew.-% polyreaktives Bindemittel,
(C) 1,5 - 20 Gew.-% besonders bevorzugt 2 - 10 Gew.-% organisches Lösungsmittel und
(D) 1 bis 30 Gew.-% weitere Hilfsmittel und Additive enthält,
jeweils bezogen auf die Gesamtmasse der Suspension.

Als Primärpartikel (A) kann gegebenenfalls eine Mischung von Primärpartikeln mit unterschiedlichen farbgebenden Komponenten verwendet werden.

Als polyreaktives Bindemittel (B) lassen sich besonders Polymerisations- und Polyadditionsharze verwenden, die in der Regel aus einer Mischung von niedermolekularen oder oligomeren Monomeren bestehen, die eine oder mehrere polyreaktionsfähige Gruppen enthalten.

Im Falle von Polymerisationsharzen sind radikalisch und kationisch polymerisierbare Harze sowie Monomere für die ringöffnende Metathesepolymerisation bevorzugt. Bei den Polyadditionsharzen sind vor allem Michael-Reaktionsharze geeignet.

Als radikalische Polymerisationsharze lassen sich insbesondere mono- oder multifunktionelle (Meth)acrylate, Mono- und Bis(meth)acrylamide oder deren Mischungen einsetzen. Dabei werden di- oder multifunktionelle Acrylate in der organischen Suspension vorzugsweise in Mischung mit Monoacrylaten eingesetzt.

Vorzugsweise werden als organisches Lösungsmittel (C) solche Komponenten eingesetzt, die eine Siedetemperatur von mindestens ca. 120 °C, vorzugsweise von 150 bis 250 °C, besonders bevorzugt von 180 bis 230 °C aufweisen, so dass es bei einer stereolithographischen Verarbeitung der Suspension nicht zu vorzeitigen Verdunstung kommt. Dabei sind Mischungen von solchen Lösungsmitteln besonders geeignet, die in einem Temperaturbereich zwischen 150 und 250 °C schrittweise verdampfbar sind. Ganz besonders geeignet sind Octanol, Triethylenglycol-Divinylether, 2-Amino-2-methyl-1-propanol, 2-Methyl-2,4-pentandiol, Ammoniumcitrat tribasisch (fest), Tripropylenglycol, Tetraethylenglycol, Triethylenglycol, Triethylcitrat, Acet-Essigsäure-Ethylester, Cyclohexanol, Cyclohexanon, Diethylenglycolmonomethylether, Oxalsäuredibutylester, 2,5-Dimethoxytetrahydrofuran, Polyethylenlycol 300, 1- oder 2-Nonanol, Diethylenglycoldiethylether, 2,5-Dimethoxytetrahydrofuran, Oxalsäuredibutylester, Cyclohexanol, Cyclohexanon, Acetessigsäureethylester und Mischungen davon.

Es wurde gefunden, dass das Verdampfen der obigen Lösungsmittel zur Bildung von Mikroporen im Grünling führt, die sich beim Sintern wieder schließen, die aber das Entweichen der Gase im Entbinderungsschritt ermöglichen und fördern und so das Entstehen von Spannungen und Rissen verhindern. Außerdem wird die Gefahr einer Trennung der stereolithographisch erzeugten Schichten reduziert und eine vollständige Entfernung der organischen Komponenten begünstigt.

Alternativ kann eine Porosität des Grünlings auch dadurch erreicht werden, dass vor Wärmebehandlung herauswaschbare Anteile extraktiv entfernt werden. Geeignete extrahierbare Komponenten sind wasserlösliche Polymere, wie z.B. Polyvinylalkohol, Polyvinylpyrrolidon und Polyethylenglycole. Weiterhin können benzinlösliche Stoffe wie Paraffine oder Wachse und langkettige Fettsäureester eingesetzt werden. Die bevorzugte Menge an extrahierbaren Komponenten in der Harzmatrix liegt zwischen 0 und 40 Gew.-%, besonders bevorzugt zwischen 0,1 und 30 Gew.-%, bezogen auf die Komponente (B).

Neben den Komponenten (A) bis (C) können die erfindungsgemäßen Suspensionen weitere Komponenten (D) als Additive enthalten.

Üblicherweise enthält die erfindungsgemäße Suspension einen Initiator, insbesondere einen Photoinitiator. Die Auswahl des Photoinitiators richtet sich nach dem verwendeten Monomertyp. Suspensionen auf der Basis von radikalisch polymerisierbaren Harzen lassen sich mit den bekannten radikalischen Photoinitiatoren für den sichtbaren Bereich (vgl. J.P. Fouassier, J.F. Rabek (Hrsg.), Radiation Curing in Polymer Science and Technology, Vol. II, Elsevier Applied Science, London und New York 1993), wie z.B. Acyl- der Bisacylphosphinoxide, vorzugsweise mit α-Diketonen, wie 9,10-Phenanthrenchinon, Diacetyl, Furil, Anisil, 4,4'-Dichlorbenzil und 4,4'-Dialkoxybenzil, und besonders bevorzugt Campherchinon polymerisieren. Zur Beschleunigung der Initiierung werden α-Diketone vorzugsweise in Kombination mit aromatischen Aminen verwendet. Redoxsysteme, die sich besonders bewährt haben, sind Kombinationen aus Campherchinon mit Aminen, wie N,N-Dimethyl-p-toluidin, N,N-Dihydroxyethyl-p-toluidin, 4-Dimethylaminobenzoesäureethylester oder strukturverwandte Systeme. Vorzugsweise werden die Initiatoren in einer Menge von 0,001 - 1,0 Gew.-%, insbesondere 0,01 bis 1,0 Gew.-%, besonders bevorzugt 0,1 bis 1,0 Gew.-% eingesetzt, jeweils bezogen auf die Gesamtmasse der Suspension.

Weiterhin kann die Suspension zusätzlich einen Inhibitor zur Verhinderung einer spontanen Polyreaktion als Stabilisator enthalten. Die Inhibitoren bzw. Stabilisatoren verbessern die Lagerstabilität der Suspension und verhindern bei der Stereolithographie darüber hinaus eine unkontrollierte Polyreaktion in der stereolithographischen Wanne. Die Inhibitoren werden vorzugsweise in einer solchen Menge zugesetzt, dass die Suspensionen über einen Zeitraum von ca. 2-3 Jahren lagerstabil sind. Besonders bevorzugt werden die Inhibitoren in einer Menge von 0,001 bis 1,0 Gew.-%, ganz besonders bevorzugt 0,001 bis 0,50 Gew.-% eingesetzt, jeweils bezogen auf die Gesamtmasse der Suspension.

Gemäß einer weiteren bevorzugten Ausführungsform enthalten die Suspensionen einen sogenannten Entbinderungsbeschleuniger. Dieser wird vorzugsweise in einer Menge von 0 bis 20 Gew.-%, besonders bevorzugt 0,01 bis 10 Gew.-% eingesetzt, jeweils bezogen auf die Gesamtmasse der Suspension. Unter Entbindungsbeschleunigern werden Stoffe verstanden, welche das Entfernen des Bindemittels während des Entbinderungsprozesses erleichtern.

Als Entbinderungsbeschleuniger lassen sich erfindungsgemäß vorteilhaft auch Comonomere einsetzen, die zu einer Verringerung der thermischen Stabilität von Polymernetzwerken führen. Hierzu eignen sich Comonomere, die thermisch labile Gruppen enthalten, wie z.B. Peroxid-, Azo- oder Urethangruppen, welche während des stereolithographischen Prozesses in das Polymernetzwerk eingebaut werden und dann im thermischen Entbinderungsprozess den Abbau des Polymernetzwerkes beschleunigen. Ein bevorzugtes Beispiel für ein polymerisationsfähiges Peroxid ist 4,4'-Divinylbenzoylperoxid. Ein bevorzugtes Beispiel für eine polymerisationsfähige Azo-Verbindung ist der Ester aus 2-Hydroxyethylmethacrylat und der 4,4'-Azobis-(4-cyano-valeriansäure). Außerdem sind als Entbinderungsbeschleuniger Comonomere geeignet, deren Polyreaktionsprodukte thermisch leicht abbaubar sind. Für radikalische Polymerisationsharze sind Comonomere bevorzugt, die wie α-Methylstyrol eine niedrige ceiling-Temperatur Tc aufweisen. Die ceiling-Temperatur ist die Grenztemperatur, bei der die Polymerisation mit der Depolymerisation im Gleichgewicht steht, und lässt sich aus dem Quotienten der Polymerisationsenthalpie und der Polymerisationsentropie berechnen (vgl. H.-G. Elias, Makromoleküle, Bd 1, 6. Aufl., Wiley-VCH, Weinheim etc. 1999, 193ff.).

Die erfindungsgemäße Suspension kann ferner ein oder mehrere Dispergiermittel enthalten, die die Bildung von Agglomeraten und das Absetzen der Keramikpartikel verhindern. Bevorzugte Dispergiermittel sind vor allem Polymere, insbesondere Polyelektrolyte, z.B. Polycarbonsäuren oder Polycarbonsäuresalze, oder nichtionische Polymere, wie z.B. Polyethylenglycol oder Carboxymethylcellulose. Als Dispergiermittel eignen sich Polyelektrolyte, die wie z.B. Ammoniumpolycarboxylat ionische Gruppen tragen und die deshalb relativ leicht an die Oberfläche von Festkörpern adsorbieren, z.B. an Keramikpartikel. Dabei können die Polyelektrolytionen den Partikeln eine elektrische Ladung verleihen, so dass man von einer elektrosterischen Wirkung spricht. Die bevorzugte Menge an Dispergiermittel beträgt 0,1 bis 5 Gew.-%, jeweils bezogen auf die Masse der Komponente (A).

Als weitere Komponenten können die erfindungsgemäßen Suspensionen einen oder mehrere Weichmacher enthalten. Der oder die Weichmacher können ggf. verhindern, dass der Keramikgrünling nach der photochemischen Aushärtung und einem evt. Trocknen brüchig wird. Darüber hinaus sorgen Weichmacher für eine ausreichende Flexibilität des stereolithographisch hergestellten Grünkörpers. Bevorzugte Weichmacher sind Phthalate, wie z.B. Dibutyl- oder Dihexylphthalat, nichtsaure Phosphate, wie z.B. Tributyl- oder Trikresylphosphat, n-Octanol, Glycerin oder Polyethylenglykole. Weichmacher werden vorzugsweise in einer Menge von 0 bis 15 Gew.-% und besonders bevorzugt 0,1 bis 5 Gew.-% eingesetzt, bezogen auf die Masse der Komponente (B).

Weiterhin können die erfindungsgemäßen Suspensionen Komponenten enthalten, die beim Entbinderungsprozess den oxidativen Abbau der Polymermatrix fördern, wie z.B. bei Raumtemperatur stabile Peroxide, oder auch katalytisch wirksame Komponenten, die eine katalytische Entbinderung ermöglichen. Neben Peroxiden eignen sich auch andere Substanzen, die oxidierend wirken, wie z.B. Salpetersäure, oder die Oxidationsmittel abspalten oder bilden.

Die rheologischen Eigenschaften der erfindungsgemäßen Suspension werden vorzugsweise so eingestellt, dass ihre Viskosität im Bereich von 200 - 20000 Pas, besonders bevorzugt 500 - 5000 Pas liegt. Dabei ist es von Vorteil, wenn möglichst keine Fließgrenzen auftreten. Die Viskosität wird bei 23 °C mit einem Platte-Platte-Viskosimeter bestimmt.

Die Erfindung betrifft auch die Verwendung einer erfindungsgemäßen Suspension zur Herstellung von Keramikformteilen und insbesondere Dentalrestaurationen, wie z.B. Inlays, Onlays, Veneers, Kronen, Brücken oder Gerüsten.

Schließlich betrifft die Erfindung auch ein Verfahren zur Herstellung eines Keramikformteils bei dem man
(a) einen Grünling herstellt, indem man eine erfindungsgemäße Suspension durch lokales Einbringen von Strahlungsenergie unter Ausbildung der geometrischen Form des Grünlings aushärtet,
(b) den Grünling einer Wärmebehandlung zur Entfernung des Bindemittels (Entbinderung) unterwirft, um einen Weissling zu erhalten, und
(c) den Weissling sintert.

Die Herstellung des Grünlings in Schritt (a) erfolgt durch Rapid Prototyping, vorzugsweise durch Stereolithographie. Es wird durch schichtweise Strahlungshärtung einer erfindungsgemäßen Suspension, insbesondere eines fließfähigen keramischen Schlickers ein keramischer Grünling hergestellt, der in Schritt (b) entbindert wird. Hierbei wird das verwendete Bindemittel durch Erhitzen des Grünlings auf eine Temperatur von vorzugsweise 90 °C bis 600 °C entfernt, und es wird der sogenannte Weissling erhalten. Der Weissling wird in Schritt (c) zu einem dichten keramischen Formkörper gesintert. Die Sinterung des Weisslings erfolgt im Sinterofen, vorzugsweise bei einer Temperatur für Zirkondioxid von 1100 bis 1600 °C, bevorzugt 1400 bis 1500 °C, für Aluminiumoxid von 1400 bis 1800 °C, bevorzugt 1600 bis 1700 °C und für Glaskeramik von 650 bis 1100 °C, bevorzugt 700 bis 900 °C. Die nach dem erfindungsgemäßen Verfahren hergestellten Keramikformkörper zeichnen sich durch eine hohe Festigkeit und große Detailgenauigkeit aus. Die Biegefestigkeit nach ISO 6872 liegt bei Formkörpern aus ZrO₂ bei mehr als 500 MPa, insbesondere im Bereich von 800 bis 1100 MPa. Formkörper aus Al₂O₃ haben eine Biegefestigkeit von vorzugsweise über 300 MPa, insbesondere im Bereich von 500 bis 700 MPa und Formkörper aus Glaskeramik vorzugsweise über 100 MPa, insbesondere im Bereich von 150 bis 500 MPa.

Die Erfindung wird im Folgenden anhand von Ausführungsbeispielen näher erläutert.

### Beispiel 1

### Grundzusammensetzung von 3Y-TZP-Keramikschlickern

| **Komponente** | **Bsp. 1A [Gew.-%]** | **Bsp. 1B [Gew.-%]** | **Bsp. 1C [Gew.-%]** | **Bsp. 1D [Gew.-%]** |
|---|---|---|---|---|
| 3Y-TZP¹⁾ | 83,0 | 83,0 | 83,0 | 83,0 |
| Mangan(III)-acetyl-acetonat¹⁾ | 0,1 | | | |
| Eisen(III)-acetyl-acetonat¹⁾ | | 0,1 | | |
| Terbium(III)-acetyl-acetonat¹⁾ | | | 0,1 | |
| Praseodym(III)-acetylacetonat¹⁾ | | | | 0,1 |
| Triphenylolpropan-triacrylat | 3,5 | 3,5 | 3,5 | 3,5 |
| Phenoxy-diethylen-glycol-diacrylat | 3,0 | 3,0 | 3,0 | 3,0 |
| Polyethylenglycol-diacrylat | 3,99 | 3,99 | 3,99 | 3,99 |
| Octanol | 3,0 | 3,0 | 3,0 | 3,5 |
| Disperbyk | 1,0 | 1,0 | 1,0 | 1,0 |
| tert-Butylperoxy-2-ethyl-hexanonat | 0,1 | 0,1 | 0,1 | 0,1 |

| | | | | |
|---|---|---|---|---|
| ¹⁾ In Form von mit dem jeweiligen Übergangsmetallsalz beschichteten Primärpartikeln | | | | |

## Patentansprüche

1. Suspension auf Basis von Oxidkeramikmaterial, die
(A) 10 - 95 Gew.-% Primärpartikel von Oxidkeramikmaterial, wobei die Primärpartikel eine durchschnittliche Partikelgröße im Bereich von 10 bis 1000 nm aufweisen und mit einer farbgebenden Komponente beschichtet sind und wobei die Primärpartikel bezogen auf die Gesamtmasse der Primärpartikel 0,000001 bis 0,4 Gew.-% der farbgebenden Komponente enthalten,
(B) 3 - 85 Gew.-% polyreaktives Bindemittel,
(C) 1 - 80 Gew.-% organisches Lösungsmittel und
(D) 1 bis 30 Gew.-% weitere Additive enthält,
jeweils bezogen auf die Gesamtmasse der Suspension.

2. Suspension nach Anspruch 1, wobei die Primärpartikel als Oxidkeramikmaterial ein Oxidkeramikmaterial auf Basis von ZrO₂ enthalten.

3. Suspension nach Anspruch 1 oder 2, wobei die Primärpartikel als Oxidkeramikmaterial ein Oxidkeramikmaterial auf Basis von mit Y₂O₃ stabilisiertem ZrO₂ enthalten, insbesondere auf Basis von 3Y-TZP.

4. Suspension nach einem der Ansprüche 1 bis 3, wobei die Primärpartikel als farbgebende Komponente eine Übergangsmetallverbindung, insbesondere eine organische Übergangsmetallverbindung enthalten.

5. Suspension nach einem der Ansprüche 1 bis 4, wobei die Primärpartikel als farbgebende Komponente ein Acetylacetonat oder ein Carbonsäuresalz der Elemente Eisen, Cer, Praseodym, Terbium, Lanthan, Wolfram, Osmium, Terbium und Mangan enthalten, insbesondere Eisen(III)-acetat oder Eisen(III)-acetylacetonat, Mangan(III)-acetat oder Mangan(III)-acetylacetonat, Praseodym(III)-acetat oder Praseodym(III)-acetylacetonat oder Terbium(III)-acetat oder Terbium(III)-acetylacetonat.

6. Suspension nach einem der Ansprüche 1 bis 5, wobei die Primärpartikel 0,01 bis 0,2 Gew.-%, bezogen auf die Gesamtmasse der Primärpartikel, farbgebende Komponente enthalten.

7. Suspension gemäß einem der Ansprüche 1 bis 6, wobei die Primärpartikel eine durchschnittliche Primärpartikelgröße im Bereich von 50 bis 500 nm, insbesondere 100 bis 200 nm aufweisen.

8. Suspension nach einem der Ansprüche 1 bis 7, die
(A) 40 - 90 Gew.-% Primärpartikel,
(B) 5 - 40 Gew.-% polyreaktives Bindemittel und
(C) 1,5 - 20 Gew.-% organisches Lösungsmittel und
(D) 1 bis 30 Gew.-% weitere Additive enthält,
jeweils bezogen auf die Gesamtmasse der Suspension.

9. Suspension nach einem der Ansprüche 1 bis 8, die als polyreaktives Bindemittel (B) mono- oder multifunktionelle (Meth)acrylate, Mono- und Bis(meth)acrylamide oder eine Mischung davon, insbesondere eine Mischung von di- oder multifunktionelle Acrylate mit Monoacrylaten enthält.

10. Suspension nach einem der Ansprüche 1 bis 9, die als organisches Lösungsmittel (C) Octanol, Triethylenglycol-Divinylether, 2-Amino-2-methyl-1-propanol, 2-Methyl-2,4-pentandiol, Ammoniumcitrat tribasisch (fest), Tripropylenglycol, Tetraethylenglycol, Triethylenglycol, Triethylcitrat, Acet-Essigsäure-Ethylester, Cyclohexanol, Cyclohexanon, Diethylenglycolmonomethylether, Oxalsäuredibutylester, 2,5-Dimethoxytetrahydrofuran, Polyethylenlycol 300, 1- oder 2-Nonanol, Diethylenglycoldiethylether, 2,5-Dimethoxytetrahydrofuran, Oxalsäuredibutylester, Cyclohexanol, Cyclohexanon, Acetessigsäureethylester oder eine Mischung davon enthält.

11. Suspension nach einem der Ansprüche 1 bis 10, die zusätzlich einen Initiator, insbesondere einen Photoinitiator enthält.

12. Suspension nach einem der Ansprüche 1 bis 11, die zusätzlich einen Inhibitor zur Verhinderung einer spontanen Polyreaktion als Stabilisator enthält.

13. Suspension nach einem der Ansprüche 1 bis 12, die zusätzlich einen Entbinderungsbeschleuniger enthält, der aus Comonomeren, die thermisch labile Gruppen enthalten, wie z.B. Peroxid-, Azo- oder Urethangruppen, und Comonomeren, die eine niedrige ceiling-Temperatur Tc aufweisen, ausgewählt ist.

14. Suspension nach einem der Ansprüche 1 bis 13, die zusätzlich ein oder mehrere Dispergiermittel, Weichmacher, Komponenten, die beim Entbinderungsprozess den oxidativen Abbau der Polymermatrix fördern, oder katalytisch wirksame Komponenten, die eine katalytische Entbinderung ermöglichen, enthält.

15. Verwendung einer Suspension gemäß einem der Ansprüche 1 bis 14 zur Herstellung von Keramikformteilen.

16. Verwendung nach Anspruch 15, wobei das Keramikformteil eine Dentalrestauration ist, wie z.B. ein Inlay, Onlay, Veneer, eine Krone, Brücke oder ein Gerüst.

17. Verfahren zur Herstellung eines Keramikformteils bei dem man
(a) einen Grünling herstellt, indem man eine Suspension gemäß einem der Ansprüche 1 bis 14 durch lokales Einbringen von Strahlungsenergie unter Ausbildung der geometrischen Form des Grünlings aushärtet,
(b) den Grünling einer Wärmebehandlung zur Entfernung des Bindemittels unterwirft, um einen Weissling zu erhalten, und
(c) den Weissling sintert.

## Claims

1. Suspension, based on oxide-ceramic material, comprising
(A) 10-95 wt % of primary particles of oxide-ceramic material, wherein the primary particles have an average particle size in the range from 10 to 1000 nm and are coated with a chromophoric component and wherein the primary particles comprise, relative to the total mass of the primary particles, 0.000001 to 0.4 wt % of the chromophoric component,
(B) 3-85 wt % of a polyreactive binder,
(C) 1-80 wt % of an organic solvent and
(D) 1-30 wt % further additives,
in each case relative to the total mass of the suspension.

2. Suspension according to claim 1, wherein the primary particles comprise an oxide-ceramic material based on ZrO₂ as the oxide-ceramic material.

3. Suspension according to claim 1 or 2, wherein the primary particles comprise an oxide-ceramic material based on ZrO₂ stabilised with Y₂O₃ as the oxide-ceramic material, in particular based on 3Y-TZP.

4. Suspension according to one of the claims 1 to 3, wherein the primary particles comprise a transition metal compound, in particular an organic transition metal compound, as the chromophoric component.

5. Suspension according to one of the claims 1 to 4, wherein the primary particles comprise an acetylacetonate or a carboxylic acid salt of the elements iron, cerium, praseodymium, terbium, lanthanum, tungsten, osmium, terbium and manganese as the chromophoric component, in particular iron (III) acetate or iron (III) acetylacetonate, manganese (III) acetate or manganese (III) acetylacetonate, praseodymium (III) acetate or praseodymium (III) acetylacetonate or terbium (III) acetate or terbium (III) acetylacetonate.

6. Suspension according to one of the claims 1 to 5, wherein the primary particles comprise 0.01 to 0.2 wt % chromophoric component, relative to the total mass of the primary particles.

7. Suspension according to one of the claims 1 to 6, wherein the primary particles have an average primary particle size in the range from 50 to 500 nm, in particular 100 to 200 nm.

8. Suspension according to one of the claims 1 to 7 comprising
(A) 40-90 wt % primary particles,
(B) 5-40 wt % of a polyreactive binder and
(C) 1.5-20 wt % of an organic solvent and
(D) 1-30 wt % further additives,
in each case relative to the total mass of the suspension.

9. Suspension according to one of the claims 1 to 8 comprising mono or polyfunctional (meth)acrylates, mono and bis(meth)acrylamides or a mixture thereof, in particular a mixture of di or polyfunctional acrylates with monoacrylates as the polyreactive binder (B).

10. Suspension according to one of the claims 1 to 9 comprising octanol, triethylene glycol divinyl ether, 2-amino-2-methyl-1-propanol, 2-methyl-2,4-pentane diol, tribasic ammonium citrate (solid), tripropylene glycol, tetraethylene glycol, triethylene glycol, triethyl citrate, ethyl acetoacetate, cyclohexanol, cyclohexanone, diethylene glycol monomethyl ether, dibutyl oxalate, 2,5-dimethoxytetrahydrofuran, polyethylene glycol 300, 1- or 2-nonanol, diethylene glycol diethyl ether, 2,5-dimethoxytetrahydrofuran, dibutyl oxalate, cyclohexanol, cyclohexanone, ethyl acetoacetate, or a mixture thereof as the organic solvent (C).

11. Suspension according to one of the claims 1 to 10 additionally comprising an initiator, in particular a photoinitiator.

12. Suspension according to one of the claims 1 to 11 additionally comprising an inhibitor as a stabiliser for preventing a spontaneous polymerisation reaction.

13. Suspension according to one of the claims 1 to 12 additionally comprising a debinding accelerator that is selected from comonomers that comprise thermally labile groups, such as e.g. peroxide, azo or urethane groups, and from comonomers that exhibit a low ceiling temperature T_{c}.

14. Suspension according to one of the claims 1 to 13 additionally comprising one or more dispersants, plasticisers, components that promote the oxidative decomposition of the polymer matrix during the debinding process, or catalytically active components that afford a catalytic debinding.

15. Use of a suspension according to one of the claims 1 to 14 for the preparation of ceramic mouldings.

16. Use according to claim 15, wherein the ceramic moulding is a dental restoration, such as e.g. an inlay, onlay, veneer, a crown, bridge or a framework.

17. Process for the preparation of a ceramic moulding in which
(a) a green compact is prepared by curing a suspension according to one of claims 1 to 14 by the local introduction of radiation energy so as to form the geometric shape of the green compact,
(b) the green compact is subjected to a heat treatment to remove the binder, in order to obtain a white body, and
(c) the white body is sintered.

## Revendications

1. Suspension à base de matériau céramique de type oxyde, qui contient :
A) de 10 à 95 % en poids de particules primaires de matériau céramique de type oxyde, lesquelles particules primaires présentent une taille moyenne de particules de 10 à 1000 nm et sont revêtues d'un composant colorant, duquel composant colorant ces particules primaires portent une quantité qui représente de 0,000001 à 0,4 % de leur poids total,
B) de 3 à 85 % en poids d'un liant polyréactif,
C) de 1 à 80 % en poids d'un solvant organique,
D) et de 1 à 30 % en poids d'autres adjuvants,
dans chaque cas, en poids rapporté au poids total de la suspension.

2. Suspension conforme à la revendication 1, dans laquelle les particules primaires contiennent, en tant que matériau céramique de type oxyde, un matériau céramique de type oxyde à base de zircone (ZrO₂).

3. Suspension conforme à la revendication 1 ou 2, dans laquelle les particules primaires contiennent, en tant que matériau céramique de type oxyde, un matériau céramique de type oxyde à base de zircone (ZrO₂) stabilisée avec de l'oxyde d'yttrium (Y₂O₃), et en particulier à base de 3Y-TZP.

4. Suspension conforme à l'une des revendications 1 à 3, dans laquelle les particules primaires portent, en tant que composant colorant, un composé de métal de transition, et en particulier un composé organique de métal de transition.

5. Suspension conforme à l'une des revendications 1 à 4, dans laquelle les particules primaires portent, en tant que composant colorant, un acétyl-acétonate ou un sel carboxylate des éléments suivants : fer, cérium, praséodyme, lanthane, tungstène, osmium, terbium et manganèse, et en particulier, de l'acétate de fer-III, de l'acétyl-acétonate de fer-III, de l'acétate de manganèse-III, de l'acétyl-acétonate de manganèse-III, de l'acétate de praséodyme-III, de l'acétyl-acétonate de praséodyme-III, de l'acétate de terbium-III, ou de l'acétyl-acétonate de terbium-III.

6. Suspension conforme à l'une des revendications 1 à 5, dans laquelle les particules primaires portent du composant colorant en une quantité qui représente de 0,01 à 0,2 % de leur poids total.

7. Suspension conforme à l'une des revendications 1 à 6, dans laquelle les particules primaires présentent une taille moyenne de particules de 50 à 500 nm, et en particulier de 100 à 200 nm.

8. Suspension conforme à l'une des revendications 1 à 7, qui contient :
A) de 40 à 90 % en poids de particules primaires,
B) de 5 à 40 % en poids d'un liant polyréactif,
C) de 1,5 à 20 % en poids d'un solvant organique,
D) et de 1 à 30 % en poids d'autres adjuvants,
dans chaque cas, en poids rapporté au poids total de la suspension.

9. Suspension conforme à l'une des revendications 1 à 8, qui contient, en tant que liant polyréactif (B), un acrylate ou méthacrylate monofonctionnel ou polyfonctionnel, un mono-acrylamide ou mono-méthacrylamide ou un bis(acrylamide) ou bis(méthacrylamide), ou un mélange de tels composés, et en particulier un mélange d'acrylates difonctionnels ou polyfonctionnels et de monoacrylates.

10. Suspension conforme à l'une des revendications 1 à 9, qui contient, en tant que solvant organique (C), l'un des composés suivants : octanol, éther divinylique de triéthylène-glycol, 2-amino-2-méthyl-propane-1-ol, 2-méthyl-pentane-2,4-diol, citrate de triammonium (solide), tripropylène-glycol, tétraéthylène-glycol, triéthylène-glycol, citrate de triéthyle, acétyl-acétate d'éthyle, cyclohexanol, cyclohexanone, éther monométhylique de diéthylène-glycol, oxalate de dibutyle, 2,5-diméthoxy-tétrahydrofurane, polyéthylèneglycol 300, nonane-1-ol, nonane-2-ol et éther diéthylique de diéthylène-glycol, en particulier le 2,5-diméthoxy-tétrahydrofurane, l'oxalate de dibutyle, le cyclohexanol, la cyclohexanone et l'acétyl-acétate d'éthyle, ou un mélange de ces composés.

11. Suspension conforme à l'une des revendications 1 à 10, qui contient en outre un amorceur, en particulier un photoamorceur.

12. Suspension conforme à l'une des revendications 1 à 11, qui contient en outre, en tant que stabilisant, un inhibiteur servant à empêcher une polyréaction spontanée.

13. Suspension conforme à l'une des revendications 1 à 12, qui contient en outre un accélérateur de déliantage, qui est choisi parmi les comonomères qui contiennent des groupes thermolabiles, comme par exemple les groupes de type peroxyde, azo et uréthane, et les comonomères qui présentent une basse température plafond T_{pf}.

14. Suspension conforme à l'une des revendications 1 à 13, qui contient en outre un ou plusieurs agents dispersants, plastifiants, composants qui accélèrent la dégradation oxydante de la matrice polymère lors du déliantage, ou composants catalytiquement actifs qui rendent possible un déliantage catalytique.

15. Utilisation d'une suspension conforme à l'une des revendications 1 à 14 pour la fabrication de pièces moulées en céramique.

16. Utilisation conforme à la revendication 15, la pièce moulée en céramique étant une prothèse de restauration dentaire, comme par exemple une incrustation inlay ou onlay, une facette, une couronne, un bridge ou une armature.

17. Procédé de fabrication d'une pièce moulée en céramique, dans lequel :
a) on prépare un corps cru, en faisant durcir une suspension conforme à l'une des revendications 1 à 14 par irradiation locale avec mise en forme géométrique du corps cru,
b) on soumet ce corps cru à un traitement thermique pour éliminer le liant, de manière à obtenir un corps blanc,
c) et l'on fritte ce corps blanc.
